# EUROPÄISCHE PATENTSCHRIFT

(11) **EP 0 285 752 B1**
(45) Veröffentlichungstag und Bekanntmachung des Hinweises auf die Patenterteilung: **27.10.1993**
(21) Anmeldenummer: 88101008.6
(22) Anmeldetag: 23.01.1988
(51) Int. Cl.: A61K 35/78, C11B 9/02

(54) **Kamillenöle mit hohem Gehalt an natürlichen Polyinen und Verfahren zu deren Herstellung**
Camomile oils having a high natural polyine content, and process for their production
Huile de camomille à forte teneur en polyines et procédé de préparation

(30) Priorität: 13.02.1987 DE 3704519
(43) Veröffentlichungstag der Anmeldung: 12.10.1988
(73) Patentinhaber: ASTA Medica Aktiengesellschaft, D-01277 Dresden (DE)
(72) Erfinder: Carle, Reinhold, Dr., D-6074 Rödermark (DE); Isaac, Otto, Dr., D-6450 Hanau 9 (DE)

(56) Entgegenhaltungen:
- AT-B- 362 056
- DE-A- 2 402 802
- Die pharmazeutische Industrie, Band 34, Nr. 2 (1972), S. 122-127
- Deutsches Arzneibuch, 7. Ausgabe 1986, S. 92-94

## Beschreibung

Die Kamille (Chamomilla recutita Rauschert (syn. Matricaria chamomilla (L.)) wird als solche oder in Form verschiedener Zubereitungen verwendet. Die Wirkung der Kamille wird auf hydrophile Wirkstoffe, wie Flavone und Polysaccharide, und auf lipophile Wirkstoffe, die Bestandteile des ätherischen Kamillenöls sind, zurückgeführt.

Von den lipophilen Kamillenwirkstoffen sind neben dem (-)-α-Bisabolol und dem Chamazulen auch die Polyine, wie zum Beispiel der cis- und trans-En-In-Dicycloether (=Spiroether), für die Qualitätsbeurteilung von Kamille und Kamillenextrakten von Bedeutung. Die Spiroether der Kamille besitzen entzündungshemmende und spasmolytische Eigenschaften. Diese Spiroether sind jedoch, besonders in der Wärme, leicht zersetzlich.

Das ätherische Kamillenöl kann entweder durch Destillation oder durch Extraktion von Kamillendroge, das heißt den getrockneten Blütenkörbchen der Kamille, gewonnen werden.

Die Destillation ermöglicht zwar eine nahezu vollständige Gewinnung der übrigen Bestandteile des ätherischen Öls, die therapeutisch wichtigen thermolabilen Spiroether werden bei diesem Vorgang jedoch weitgehend zerstört. Das destillativ gewonnene Öl zeigt somit nicht mehr das kamillentypische Wirkstoffprofil.

Zwar wird in der Literaturstelle "Die pharmazeutische Industrie", Band 34, Nr. 2 (1972), Seiten 122-127 für Kamillenöle, die durch Wasserdampfdestillation von getrockneten Kamillenblüten (Apparatur nach DAB 7) erhalten werden ein höherer Gehalt an En-In-Dicycloether (Cis- und trans Spiroether) angegeben, wobei dieser Gehalt gaschromatographisch mittels eines Flammenionisationsdetektors bestimmt wurde; hierbei wurde jedoch kein En-In-Dicycloether als Referenzsubstanz verwendet. Ohne die gleichzeitige Verwendung der jeweils reinen Substanz als Referenz erlaubt die Gehaltsbestimmung mittels dem Flammenionisationsdetektor jedoch keine Rückschlüsse auf den genauen Gehalt (Menge) der betreffenden Substanz (das Anzeig-Signal für den Gehalt an einer bestimmten Substanz ist abhängig von der Oxydationsstufe der betreffenden Substanz). Die in dieser Literaturstelle angegebenen Mengenwerte für die erhaltenen En-In-Dicycloether sind daher viel zu hoch. Die Verwendung frischer Blüten oder von Extraktionsrückständen für die Herstellung eines Kamillenöls geht aus dieser Stelle nicht hervor.

Andererseits gelingt es auch nicht, das ätherische Kamillenöl durch Extraktion vollständig zu gewinnen. So geht beispielsweise bei der Herstellung von Fluidextrakten mit 45 %igem Ethanol nur etwa die Hälfte des in der Droge enthaltenen bisabolol- und chamazulenhaltigen Öls mit den empfindlichen Spiroethern in den Extrakt über.
Es wurde nun gefunden, daß bei der Wasserdampfdestillation von Kamillenextraktionsrückständen (Rückstände von Drogenextraktionen oder von Frischkamilleextraktionen) oder von frischen Kamillenblüten (Frischkamille) Kamillenöle erhalten werden, die trotz der thermischen Behandlung hohe Gehalte an Polyinen, das heißt insbesondere hohe Gehalte an thermolabilem cis- beziehungsweise trans-En-In-Dicycloether aufweisen.

Es ist dabei in höchstem Maße überraschend und nicht naheliegend, daß es möglich ist, aus den Rückständen von Kamillenextraktion, die bislang als wertlos verworfen wurden, nochmals ein wertvolles Kamillenöl mit hohen Gehalten an En-In-Dicycloether zu gewinnen. Ebenso überraschend ist die Gewinnung eines Kamillenöls mit sehr viel höherem Gehalt an den genannten Dicycloethern bei Verwendung von Frischkamille an Stelle von getrockneten Kamillenblüten (Droge) wie das Beispiel 2 zeigt.

Der cis-En-In-Dicycloether (Auch cis-Spiroether genannt) hat die folgende Struktur: 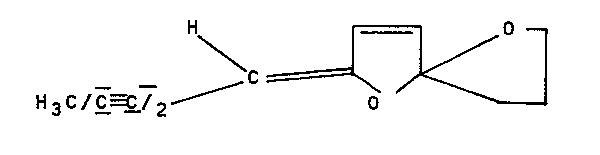
Der trans-En-In-Dicycloether (trans-Spiroether) hat die folgende Struktur: 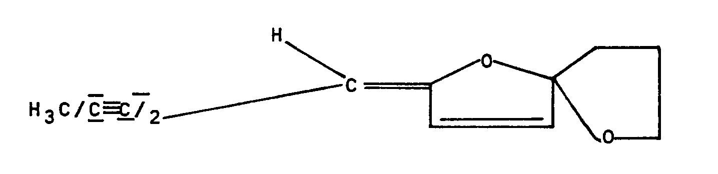
Das erfindungsgemäße Verfahren erlaubt daher erstmalig die Herstellung von Kamillenöl mit dem spezifischen Wirkstoffprofil der Kamille. Ein solches Kamillenöl ist beispielsweise als Zusatz zu wäßrig-alkoholischen Kamillenextrakten geeignet, um diese auf einen hohen Gehalt an ätherischem Öl mit den lipophilen Kamillenwirkstoffen zu standardisieren, wobei nun jedoch das typische Wirkstoffprofil der Kamille erhalten bleibt.

Darüberhinaus ermöglicht das erfindungsgemäße Verfahren die wirtschaftliche Verwertung von Rückständen aus der Kamillenextraktion, wobei das noch in den Extraktionsrückständen der Kamillendroge oder der Frischkamille enthaltene ätherische Öl mit seinen Wirkstoffen unverändert zurückgewonnen wird. Dies führt neben der besseren Wirkstoffausbeute zu erheblichen Einsparungen an Rohstoffen für die Extraktherstellung.

Bei der Destillation der Extraktionsrückstände frischer Kamillenblüten oder von unbearbeiteten frischen Kamillenblüten wird nicht nur die Benutzung kostenaufwendiger Trocknungsanlagen überflüssig. Auch die erheblichen Betriebskosten für die übliche Vorbereitung des Destillationsguts durch Trocknung entfallen. Die Kosten für die Herstellung von Extraktionspräparaten lassen sich auf diese Weise deutlich senken.

Ein Zusatz erfindungsgemäß gewonnener Kamillenöle mit hohem Gehalt an Spiroethern führt auch im Gemisch mit herkömmlich aus Kamillendroge destillierten Kamillenölen bei der Herstellung von Kamillenextraktpräparaten zu Extrakten mit kamillentypischem Wirkstoffprofil.

Das erfindungsgemäße Verfahren erfolgt mittels Wasserdampfdestillation oder durch Wasserdestillation.

Bei der Wasserdampfdestillation wird Heißdampf in ein Destillationsgefäß eingeleitet. Der Heißdampf kann unter Normaldruck oder unter Überdruck bis zu 4 - 5 bar stehen.

Beispielsweise kann der Heißdampf unter einem Druck von 0-1, vorzugsweise 0-0,5 bar stehen. Die Temperatur des Heißdampfes liegt beispielsweise zwischen 100 bis 140 °C (maximal 145 °C), vorzugsweise 100 bis 130 °C, insbesondere 105 bis 115 °C.
Die Destillationsdauer beträgt beispielsweise 2 - 10 Stunden, vorzugsweise 2 - 4 Stunden. Bei der Wasserdampfdestillation werden dem Destillationsgut keine Zusätze zugegeben. Die Destillation kann kontinuierlich oder diskontinuierlich erfolgen.

Die Wasserdestillation erfolgt durch Erhitzen eines mit Wasser beschickten Destillationsgefäßes (Temperatur: beispielsweise 100 °C). Dabei wird ein Gewichtsteil Destillationsgut mit mindestens einem Gewichtsteil Wasser, vorzugsweise mit 10-100 Gewichtsteilen, insbesondere mit 10-60 Gewichtsteilen Wasser versetzt, das übliche Zusätze, wie reduzierende Mittel (zum Beispiel Natriumascorbat, Ascorbinsäure sowie Mineralsäuren (wie Salzsäure, Schwefelsäure) oder Alkalihydroxyde (wie verdünnte NaOH) zur pH-Einstellung enthalten kann. Die Destillation kann bei pH-Werten zwischen 4-8 erfolgen; vorzugsweise wird auf eine Einstellung des pH verzichtet, so daß bei neutralem pH destilliert wird. Die Wasserdestillation wird nach 1-4 Stunden, vorzugsweise nach 2-3 Stunden beendet.
Die Menge an zugesetzten reduzierenden Mitteln beträgt beispielsweise 0,1 bis 1 Gewichtsteile bezogen auf 1 Gewichtsteil Pflanzenmaterial.

Zur Durchführung der Destillation kann das Destillationsgut zerkleinert verwendet werden. Der Feuchtigkeitsgehalt des eingesetzten Destillationsgutes liegt beispielsweise bei Verwendung von Rückständen aus Kamillenextraktionen im Durchschnitt bei 60 % (Wasser-Alkohol), bei Verwendung von Frischkamille im Durchschnitt bei 80 % (Wasser). Die Destillation kann in den üblichen mobilen oder stationären Destillationsbehältern vorgenommen werden.

Das durch Kühlung kondensierte Destillat wird beispielsweise in einer Vorrichtung (Gefäß) aufgefangen, die eine Phasentrennung des ätherischen Kamillenöls und Wasser aufgrund der unterschiedlichen spezifischen Dichte ermöglicht (zum Beispiel Florentiner Flasche, aber auch andere übliche Separatoren sowie Separatoren mit Zentrifugiereinrichtungen).
Das ätherische Kamillenöl der Oberphase kann beispielsweise nach Abtrennung von der Wasserphase ohne weitere Reinigung zur Herstellung von Kamillenextraktpräparaten verwendet werden. Die Phasentrennung kann beispielsweise durch Zusatz von Salzen (zum Beispiel NaCl) erleichtert werden (Aussalzen).

Bei der Wasserdestillation kann gegebenenfalls die Destillationsanlage nach Beendigung der Destillation mit einem niedrigsiedenden lipophilen Lösungsmittel, zum Beispiel einem gesättigten flüssigen aliphatischen Kohlenwasserstoff mit 5 bis 7 C-Atomen (zum Beispiel n-Pentan, Petrolether) ein- bis mehrmals (2 bis 3 mal) durchgespült werden. Diese Spülung dient der Entfernung letzter Ölreste aus der Destillationsbrücke. Für die Spülung werden zum Beispiel 1 bis 2 Gewichtsteile Kohlenwasserstoff pro 1 Gewichtsteil Trockengewicht des verwendeten Extraktionsrückstandes verwendet. Nach Entfernen des Kohlenwasserstoffs (siehe unten) wird der Rückstand mit dem Kamillenöl des wäßrigen Destillats vereinigt.

Falls der Kohlenwasserstoff mit dem wäßrigen Destillat vereinigt wird, gehen die Wirkstoffe des Destillats in den Kohlenwasserstoff. Abtrennung der Kohlenwasserstoffphase von der wäßrigen Phase erfolgt dann beispielsweise in bekannter Weise durch Zugabe von NaCl (Aussalzen) gefolgt von wiederholtem Ausschütteln (zum Beispiel 2 mal) der wäßrigen Phase mit dem Kohlenwasserstoff. Nach Trocknen der Kohlenwasserstoffphase wird das Kamillenöl durch Entfernen des Kohlenwasserstoffs, zweckmäßig bei Temperaturen zwischen 40 und 60 °C, erhalten. Die letzten Reste von Kohlenwasserstoff können zum Beispiel mittels eines Warmluftstroms (Temperatur 40 bis 60 °C) entfernt werden.

Unter frischen Kamillenblüten werden Kamillenblüten verstanden, die innerhalb von 24 Stunden nach dem Pflücken destilliert werden oder innerhalb dieser Frist eingefroren werden.

Das erfindungsgemäße Verfahren ist ganz allgemein anwendbar, das heißt es eignet sich für alle vorkommenden Kamillen, welche Spiroether enthalten. Insbesondere werden beispielsweise folgende Kamillen (beziehungsweise die daraus hergestellten Drogen) verwendet: Die diploide Kamillensorte Degumill (DDR-Sortenschutzrecht Degumill; Deutsches Patent 24 02 802; Italienisches Patent 1 035 096), die tetraploide Kamillensorte Manzana (Deutsche Offenlegungsschrift 34 23 207), tetraploide Kamillen gemäß der deutschen Offenlegungsschrift 35 42 756.

Weiterhin kommen zum Beispiel folgende diploide Ausgangskamillen in Frage: "Kamille argentinischer Provenienz" (siehe L.Z. Padula, R.V.D. Rondina und J.D. Coussio, Quantitative Determination of Essential Oil. Total Azulenes and Chamazulene in German Chamomile, Matricaria chamomilla, Cultivated in Argentinia; Planta med. 30, Seiten 273-280, 1976) sowie alle Kamillen, welche im ätherischen Öl deutlich meßbare Kozentrationen an (-)-α-Bisabolol (in der Regel über 5 % des ätherischen Öls) aufweisen. Beispielsweise kommen solche diploiden Kamillen in Frage, die in folgenden Literturstellen beschrieben sind: Schilcher, H., "Neuere Erkenntnisse bei der Qualitätsbeurteilung von Kamillenöl beziehungsweise Kamillenblüten", Planta med. 23, 132-144 (1973); Motl, O., M. Felklová; V. Lukes & M. Jasicová "Zur gaschromatographischen Analyse und zu chemischen Typen von Kamillenöl", Arch. Pharm. 310, 210-215 (1977); Franz, Ch., J. Hölzl & A. Vömel "Preliminary Morphological and Chemical Characterization of some Populations and Varieties of Matricaria chamomilla L.", Acta Hort. 73, 109-114 (1978).
Folgende tetraploide Ausgangskamillen kommen zum Beispiel ebenfalls in Frage: Die Kamillensorten Bodegold (DDR), Pohorelicky (CSSR), Zloty Lan (Polen), BK-2 (Ungarn). Diese Sorten sind in den folgenden Literaturstellen beschrieben: M. Chládek, V. Kosova, K. Hruby, Pharmazie 13, 712-718 (1958); W. Czabajska, Diss. Posna 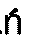 (1963); W. Poethke und P. Bulin, Pharm. ZHalle 108, 813-823 (1969); I. Sárkány, Herb. Hungar. 4 (1), 125-169 (1965).

Als Kamillenextraktionsrückstände, die für das erfindungsgemäße Verfahren eingesetzt werden, kommen solche Rückstände in Frage, die ganz allgemein bei der üblichen Extraktion von Kamillendrogen oder von Frischkamille mit Alkoholen beziehungsweise Alkohol-Wasser-Gemischen erhalten werden. Beispielsweise handelt es sich bei den für solche Extraktionen verwendeten Alkoholen um folgende Alkohole und deren Mischungen mit Wasser: Gerade oder verzweigte aliphatische Alkohole mit 1 - 6 Kohlenstoffatomen sowie auch Solketal (2,2-Dimethyl-4-hydroxymethyl-1,3-dioxolan), wie zum Beispiel Methanol, Ethanol, Propanol-(1), Propanol-(2), Butanol und ähnliche. Es können auch Gemische dieser Lösungsmittel sowie Mischungen mit Wasser verwendet werden. Im Falle wäßriger Mischungen beträgt der Wassergehalt zum Beispiel 0 bis 65, vorzugsweise 20 bis 60, insbesondere 30 bis 55 Volumenprozent (Volumen in Volumen).

Die Kamillenextraktionsrückstände können sofort, das heißt noch im feuchten Zustand, der erfindungsgemäßen Destillation unterworfen werden.
Es ist aber auch möglich, diese Rückstände zu trocknen (Bedingungen: 40-60 °C, 2-5 Stunden) und gegebenenfalls nach Lagerung (Bedingungen: 15-25 °C, relative Luftfeuchtigkeit: 40-50 %) und Transport erfindugsgemäß weiter zu verarbeiten.

Das nach dem erfindungsgemäßen Verfahren erhaltene Kamillenöl enthält mindestents 1 Gewichtsprozent, vorzugsweise mindestens 1,5, insbesondere mindestens 2 Gewichtsprozent an Polyinen (Summe aller Polyine).

Beispielsweise ist der Gehalt an Polyinen (Summe aller Polyine, wobei es sich um natürliche Polyine handelt) 2 bis 20, vorzugsweise 4 bis 18, insbesondere 10 bis 15 Gewichtsprozent. Der Gehalt an cis-Spiroether ist beispielsweise mindestens 1 Gewichtsprozent, vorzugsweise 2 bis 18, insbesondere 5 bis 15 Gewichtsprozent. Der Gehalt an trans-Spiroether ist beispielsweise mindestens 0,5, vorzugsweise 0,5 bis 15, insbesondere 1 bis 10 Gewichtsprozent. Der Gehalt an Chamazulen liegt beispielsweise zwischen 0,9 und 8 Gewichtsprozent, vorzugsweise zwischen 3 und 6 Gewichtsprozent. Der Gehalt an α-Bisabolol liegt beispielsweise zwischen 5 und 30 Gewichtsprozent, vorzugsweise zwischen 22 und 32 Gewichtsprozent. Der Gehalt an Bisabololoxid A liegt beispielsweise zwischen 1 und 10 Gewichtsprozent, vorzugsweise zwischen 2 und 4 Gewichtsprozent. Der Gehalt an Bisabololoxid B liegt beispielsweise zwischen 0,8 und 15 Gewichtsprozent, vorzugsweise zwischen 2 und 4 Gewichtsprozent.

Das nach dem erfindungsgemäßen Verfahren gewonnenen Kamillenöl wird beispielsweise in reiner Form oder als Lösung in einem physiologisch verträglichen Lösungsmittel (zum Beispiel Ethanol) wäßrig-alkoholischen Kamillenextrakten, die in bekannter Weise erhalten werden, unter mischen zugesetzt. Bei den wäßrigalkoholischen Extrakten handelt es sich beispielsweise um solche, die unter Verwendung von Ethanol oder Isopropanol hergestellt werden. Der Alkoholgehalt soll 20 Gewichtsprozent nicht unterschreiten. Im allgemeinen soll der Alkoholgehalt des Extraktes 20 bis 55, vorzugsweise 20 bis 50 Gewichtsprozent, insbesondere 20 bis 40 Gewichtsprozent betragen.

Der Zusatz des erfindungsgemäßen Kamillenöls zu solchen Extrakten ist so zu bemessen, daß im Endprodukt ein Gehalt an ätherischem Öl von 100-250 mg pro 100 g, vorzugsweise 180-200 mg pro 100 g alkholischem beziehungsweise wäßrig-alkoholischem Extrakt resultiert.

Der Bisabololgehalt beträgt dann beispielsweise bei Verwendung einer bisabololreichen Kamille 20-100 mg pro 100 g, vorzugsweise 40-80 mg pro 100 g Extrakt. Der Extrakt enthält dann beispielsweise 1,5 mg pro 100 g cis- und trans-Spiroether.
Kamillenextrakte oder Kamillenextraktpräparate, die unter Zusatz des erfindungsgemäßen Kamillenöls erhalten werden, enthalten beispielsweise:
mindestens 1,0 mg pro 100 g Polyine, vorzugsweise 1 bis 10, insbesondere 2 bis 5 mg pro 100 g. Der Gehalt an cis-Spiroether ist mindestens 0,5 mg pro 100 g, vorzugsweise 0,7 bis 4,0, insbesondere 1,0 bis 3,0 mg pro 100 g. Der Gehalt an trans-Spiroether ist mindestens 0,3, vorzugsweise 0,7 bis 3,0, insbesondere 0,7 bis 1,4 mg pro 100 g. Der Gehalt an Chamazulen liegt beispielsweise zwischen 3 und 25 mg pro 100 g, vorzugsweise zwischen 3 und 20, insbesondere zwischen 3 und 18 mg pro 100 g. Der Gehalt an α-Bisabolol liegt beispielsweise zwischen 30 und 100 mg pro 100 g, vorzugsweise zwischen 30 und 80, insbesondere zwischen 40 und 60 mg pro 100 g. Der Gehalt an Bisabololoxid A liegt beispielsweise zwischen 5 und 30 mg pro 100 g, vorzugsweise zwischen 5 und 25, insbesondere zwischen 5 und 20 mg pro 100 g. Der Gehalt an Bisabololoxid B liegt beispielsweise zwischen 3 und 20 mg pro 100 g, vorzugsweise zwischen 3 und 18, insbesondere zwischen 3 und 15 mg pro 100 g.

### Beispiel 1

41 g Kamille-Extraktionsrückstand* (entsprechend 10 g Trockengewicht) werden in einem 1000-ml-Rundkolben mit 500 ml Wasser versetzt, die Mischung mit verdünnter Natriumhydroxid-Lösung auf einen pH-Wert von 6,5 eingestellt und etwa 3 Stunden lang destilliert. Als Auffanggefäß dient zum Beispiel ein Schütteltrichter von 1000 ml Inhalt. Nachdem 400 ml Destillat erhalten worden sind, wird ohne Kühlung etwa 2 Minuten lang weiterdestilliert. Anschließend werden Destillationsbrücke und Kühler mit 80 ml Pentan durchgespült. Das Pentan wird mit dem Destillat vereinigt und nach Zugabe von 65 g Natriumchlorid ausgeschüttelt. Der Ausschüttelvorgang wird noch 2mal mit je 80 ml Pentan wiederholt. Die vereinigten organischen Phasen werden über 8 g wasserfreiem Natriumsulfat getrocknet und filtriert. Vorlage und Filter werden 2mal mit je 10 ml Pentan nachgewaschen und die Pentanphase anschließend bis auf etwa 5 ml schonend eingeengt (Wasserbad 50 °C, Rotationsverdampfer). Der Rest des Pentan wird durch einen Warmluftstrom (Temperatur: 50-60 °C, Fön) abgeblasen.

Es resultiert ein Kamillenöl folgender Zusammensetzung:

| | |
|---|---|
| Chamazulen | 3,6 % |
| (-)-α-Bisabolol | 20,5 % |
| Bisabololoxid A | 2,7 % |
| Bisabololoxid B | 4,1 % |
| cis-Spiroether | 10,2 % |
| trans-Spiroether | 1,7 % |
| *Es handelt sich um den Kamillenrückstand einer mit Ethanol-Wasser (47:53 Volumen in Volumen) durchgeführten Kamillen-Extraktion. Die eingesetzte Kamillendroge war eine bisabololreiche Droge, die aus der Kamillensorte Degumill gemäß der deutschen Patentschrift 24 02 802 hergestellt worden war. | |

### Vergleichsbeispiel (bekanntes Verfahren)

10 g getrocknete Kamillenblüten (die Droge ist identisch mit der im Beispiel 1 verwendeten Kamillendroge vor der Extraktion) werden, wie unter Beispiel 1 beschrieben, destilliert.

Das abdestillierte Öl zeigt folgende Zusammensetzung:

| | |
|---|---|
| Chamazulen | 7,6 % |
| (-)-α-Bisabolol | 24,4 % |
| Bisabololoxid A | 1,6 % |
| Bisabololoxid B | 1,7 % |
| cis-Spiroether | 0,2 % |
| trans-Spiroether | nicht nachweisbar. |

### Beispiel 2

37,8 g bisabololreiche Frischkamille der Kamillensorte Degumill (entsprechend 10 g Droge, das heißt getrockneten Kamillenblüten) werden in einen 1000-ml-Zweihalskolben eingebracht. Nach Aufsetzen der Destillationsbrücke mit Kugelkühler wird durch den seitlichen Ansatz Wasserdampf aus einer Dampfkanne eingeleitet. Die Destillation wird nach 4 Stunden abgebrochen.

Das abdestillierte ätherische Öl der Oberphase wird mittels einer Florentiner Flasche abgetrennt und die Zusammensetzung des Öls analysiert.

| | |
|---|---|
| Chamazulen | 12,5 % |
| (-)-α-Bisabolol | 22,1 % |
| Bisabololoxid A | 4,7 % |
| Bisabololoxid B | 3,4 % |
| cis-Spiroether | 11,8 % |
| trans-Spiroether | 7,3 % |

### Vergleichsbeispiel (bekanntes Verfahren)

10 g getrocknete Kamillenblüten (die Droge ist identisch mit der im Beispiel 2 verwendeten, schonend getrockneten Kamille) werden, wie unter Beispiel 2 beschrieben, destilliert.

Das aus der Oberphase abgetrennte Kamillenöl weist folgende Zusammensetzung auf:

| | |
|---|---|
| Chamazulen | 9,6 % |
| (-)-α-Bisabolol | 21,2 % |
| Bisabololoxid A | 3,0 % |
| Bisabololoxid B | 3,9 % |
| cis-Spiroether | 1,2 % |
| trans-Spiroether | 0,5 %. |

## Patentansprüche

1. Verfahren zur Herstellung von Kamillenölen mit einem Gehalt an natürlichen Spiroethern, von mindestens 1 Gewichtsprozent dadurch gekennzeichnet, daß man Frischkamllle oder Extraktionsrückstände von Kamillenextraktionen entweder einer Wasser-dampfdestillation oder einer Destillation mit Wasser unterwirft.

2. Verfahren nach Anspruch 1,
dadurch gekennzeichnet,
daß die Wasserdestillation bei einem pH-Wert zwischen 4 bis 8, gegebenenfalls unter Zusatz von reduzierenden Stoffen, durchgeführt wird.

3. Verfahren zur Herstellung von Kamillenöl nach einem oder mehreren der vorangegangenen Ansprüche 1 und 2,
dadurch gekennzeichnet,
daß der Gehalt an natürlichem cis-Spiroether mindestens 1 und an natürlichen trans-Spiroether mindestens 0,5 Gewichtsprozent beträgt.

## Claims

1. A process for the preparation of camomile oils containing at least 1 percent by weight of natural spiro ethers, characterized in that fresh camomile or extraction residues from camomile extractions are subjected either to steam distillation or to distillation with water.

2. A process according to Claim 1, characterized in that the distillation with water is carried out at a pH of between 4 and 8, optionally with the addition of reducing substances.

3. A process for the preparation of camomile oil according to one or more of the preceding Claims 1 and 2, characterized in that the content of natural cis-spiro ether is at least 1 and the content of natural trans-spiro ether is at least 0.5 percent by weight.

## Revendications

1. Procédé de préparation d'huiles de camomille avec une teneur en ène-yne-dicycloéthers naturels, d'au moins 1% en poids, caractérisé en ce que l'an soumet de la camomille fraîche ou des résidus d'extraction provenant d'extractions de camomille à un entraînement à la vapeur ou à une distillation avec de l'eau.

2. Procédé selon la revendication 1, caractérisé en ce que la distillation d'eau est effectuée à une valeur de pH comprise entre 4 et 8, éventuellement avec addition de produits réducteurs.

3. Procédé de préparation d'huile de camomille selon la revendication 1 et/ou la revendication 2, caractérisé en ce que la teneur en cis-ène-yne-dicycloéther naturel est d'au mains 1% en poids et la teneur en trans-ène-yne-dicycloéther naturel est d'au mains 0,5%, en poids.
